# EUROPEAN PATENT APPLICATION

(11) **EP 4 066 883 A1**
(43) Date of publication of application: **05.10.2022**
(21) Application number: 21165637.6
(22) Date of filing: 29.03.2021
(51) Int. Cl.: A61N 1/36, A61N 1/372

(54) **SYSTEM, IMPLANT UNIT AND METHOD FOR THE TREATMENT OF HEAD AND FACIAL PAIN**

(71) Applicant: Man & Science S.A., Mont-Saint-Guibert (BE)
(72) Inventor: Van Buyten, Jean-Pierre, Mont-Saint-Guibert (BE); Mével, Hervé, Mont-Saint-Guibert (BE); Taub, Robert, Mont-Saint-Guibert (BE)
(74) Representative: Patentanwälte Olbricht Buchhold Keulertz

(57) **Abstract**

Disclosed herein is a neurostimulation system for treating head pain, the system comprising: an implant unit configured for implantation inside a subject's (700) body; an external unit configured for a location external to the subject's (700) body; and a charging and programming unit; wherein the external unit comprises: a processor; a power source (220); and a primary transmission unit (230) in electrical communication with the power source (220) and the processor; wherein the implant unit comprises: at least one lead (310); at least one pair of modulation electrodes (320) attached to the at least one lead (310); and a secondary transmission unit (330) in electrical communication with the at least one lead (310); and wherein the processor is configured to establish a coupling between the primary transmission unit (230) and the secondary transmission unit (330) and to transmit power from the power source (220) to the implant unit via said coupling. According to another aspect of the present disclosure, an implant unit for use in a neurostimulation system is described, wherein the implant unit is configured for implantation inside a subject's (700) body through an incision (600) subject's (700) skin, and wherein the implant unit is further configured for implantation inside the subject's (700) body through a tunnel (610). According to yet another aspect of the disclosure, a method for electrical stimulation of neuromuscular tissue using a neurostimulation system is described herewith, the method comprising: generating an electrical stimulation pattern with an external unit, the electrical stimulation pattern comprising at least one modulation signal; delivering the electrical stimulation pattern to an implant unit located inside a subject's (700) body; adjusting the electrical stimulation pattern, wherein adjusting the electrical stimulation pattern comprises increasing or decreasing a voltage, a current amplitude, a pulse frequency and/or a pulse width of the electrical stimulation pattern. Such a method may be valuable, for example, in pain management, where the propagation of pain signals is undesired.

## Description

### Technical Field

The disclosed subject matter described hereafter refers to a system and a method for electrical nerve stimulation. Furthermore, reference is made to a use of the method for electrical nerve stimulation in the treatment of head and facial pain.

### Background

Neural modulation, i.e. electrical stimulation of nerves, presents the opportunity to treat many physiological conditions and disorders by interacting with the body's own natural neural processes. Neural stimulation includes inhibition (e.g. blockage), modulation, modification, regulation, or therapeutic alteration of activity, electrical or chemical, in the central, peripheral, or autonomic nervous system.

By modulating the activity of the nervous system, for example through the stimulation of nerves or the blockage of nerve signals, several different goals may be achieved. Motor neurons may be stimulated at appropriate times to cause muscle contractions. Sensory neurons may be blocked, for instance to relieve pain, or stimulated, for instance to provide a signal to a subject. In other examples, modulation of the autonomic nervous system may be used to adjust various involuntary physiological parameters, such as heart rate and blood pressure. Neural modulation may provide the opportunity to treat several diseases or physiological conditions, a few examples of which are described in detail below.

For the purpose of this disclosure, the terms "stimulation" and "modulation" are used interchangeably if nothing else is specified.

Typically, neural stimulators deliver therapy in the form of electrical pulses and include two or more electrode in a proximity of the target location, such as a specific nerve or section thereof. Electrical stimulation is adjustable through various parameters, such as the polarity of electrode(s), voltage or current amplitudes, pulse frequency, pulse width, configuration and selection of electrodes and others, as these define the electrical stimulation therapy to be delivered to the user in need of therapy. Such parameters may be preprogrammed or programmable to deliver the desired stimulation and result desired from the stimulation therapy.

Among the conditions to which neural stimulation may be applied, is the occurrence of migraine headaches. Conventional treatments typically involve the use of analgesics of varying strengths. However, due to neural involvement in the sensation of pain, methods and apparatuses aimed at neural stimulation may offer a different solution. Pain sensation in the head is transmitted to the brain via afferent or sensory neurons. Such neurons may include the greater occipital nerve, the lesser occipital nerve, the third occipital nerve and the trigeminal nerve. When a subject experiences head and facial pain, such as during a migraine headache, the inhibition of these nerves may serve to decrease or eliminate the sensation of pain.

Neural stimulation may also be an effective solution to other conditions, for example, cluster headaches, or even sleep disordered breathing and hypertension. The foregoing are just a few examples of conditions to which neuromodulation may be of benefit, however embodiments of the invention described hereafter are not necessarily limited to treating only the above-described conditions.

### Prior Art

Known implantable systems for neuromodulation are usually made up of three parts: a lead, a rechargeable or non-rechargeable implantable pulse generator, and extensions to connect the pulse generator and the lead. These extensions are required for the delivery of electrical stimulation to the occipital nerves, as the pulse generator is implanted in the abdomen or in the flank. However, this technology has several disadvantages. For example, it leads to insufficient coverage of pain area in some patients. In addition, implantation of such large devices requires a long and invasive surgical procedure. Another drawback comes from the fact that the excess need for tunneling and extension leads to possible infection, dislocation, lead breakage and erosion risks. Lastly, the non-rechargeable implanted pulse generator creates the need for revision with the consequence of a higher revision rate and higher cost.

### Summary of the Disclosed Subject-matter

One of the objectives of the present disclosure is to respond to the disadvantages of the prior art and to provide an improved system for electrical nerve stimulation. In particular, a system should be provided that allows for a simple and time-efficient implantation procedure, improving the results by new technology and implant technique and avoiding the burdens of existing technologies.

According to one aspect of the disclosure, a neurostimulation system for treating head and facial pain is provided, the system comprising an implant unit configured for implantation inside a subject's body; an external unit configured for a location external to the subject's body; wherein the external unit comprises: a processor, a power source and a primary transmission unit in electrical communication with the power source and the processor; wherein the implant unit comprises: at least one lead, at least one pair of modulation electrodes attached to the at least one lead, and a secondary transmission unit in electrical communication with the at least one lead and its electronics; and wherein the processor of the external unit is configured to establish a coupling between the primary transmission unit and the secondary transmission unit and to transmit power from the power source to the implant unit via said coupling.

The implant unit may preferably comprise only basic circuitry in order to perform the desired neurostimulation tasks. Advantageously, this leads to the implant unit not requiring its own energy or power reservoir embedded in its housing. Instead, power supply may for example be achieved via an inductive transcutaneous radiofrequency link (RF) between the external unit and the implant unit or between the physician programmer and the implant unit by direct RF transmission (i.e. BLE - Bluetooth Low Energy). According to further embodiments, the implant unit may comprise, enclosed in a sealed housing, one or more of the following: a secondary transmission unit or antenna for receiving and/or sending one or more signals from an external primary transmission unit; an AC/DC converter; a CPU or a memory (e.g. a very low power microcontroller); at least on current source; a stimulation lead comprising at least one modulation electrode; a programmable stimulation profile look-up table; a bootloader embedded software; an analog multiplexer to configure the modulation electrodes connected to the voltage or current source.

Amongst the preferred functions of the presented implant unit may be the following: run neurostimulation therapy according to a stimulation profile stored in the lookup table; regularly run diagnostics of both the patient and the implant unit on; send data or updates on the patient's and/or the implant unit's status to an external unit when an inductive transmission channel between the implant unit and the external is established.

Preferably, the external unit is rectangular-, circular- or oval-shaped and may be attached to an external carrier. In an even more preferred embodiment, the external unit is ergonomically designed to fit behind a subject's ear. The external unit may further comprise a housing, wherein the housing can contain the processor, the primary transmission unit and the power source. Examples for the external unit include patches, buttons, ear pieces or other receptacles. In one embodiment, for example, the housing of the external unit may include a flexible material such that the external unit may be configured to conform to a desired location. According to certain embodiments, the external unit may, encapsulated in a housing, further comprise one or more of the following: a primary transmission unit or antenna for receiving from and/or sending to the internal secondary transmission unit one or more inducing signals; an DC/AC converter; a power source; a CPU or a memory (e.g. a microcontroller); a bicolor LED (e.g. for emitting red and/or green light); a vibrator module for giving feedback to the patient in case the primary and secondary antennae are aligned with each other; a demodulator receiving implant unit status as well as alerts and warnings sent from the implant unit; a push button.

Amongst the preferred functions of the external unit may be the following: provide power to supply the implant unit via inductive coupling; recharge the battery located in the external unit when placed on a charging unit; receive data from the implant unit when an inductive transmission channel between the implant unit and the external is established; transfer said data to a charging unit; power a bicolor LED; etc.

In a preferred embodiment, the primary transmission unit may be a coil antenna. The coil may be made from any suitable conductive material and may be configured to include any suitable arrangement of conductive coils (diameter, number of coils, layout of coils, etc.). A coil suitable for use as primary transmission unit may have a diameter of about 0,5 cm to 10 cm, and may be circular-shaped, rectangular-shaped or oval-shaped. In some embodiments, the coil antenna may have a diameter of about 0,5 cm to 2,5 cm. A coil antenna suitable for use as primary transmission unit may have any number of windings. Further, a coil antenna suitable for use as primary transmission unit may have a wire diameter of about 0.1 mm to 2 mm. According to another embodiment, the transmission unit can be a printed circuit board antenna or it can be directly printed on the housing of the external unit.

The external carrier may be configured for attachment to the subject's skin. For example, the carrier may be a flexible skin patch configured for adherence to the subject's skin, e.g. through adhesives or mechanical means. It is also possible to attach the external unit to the subject's skin via a magnetic force, wherein the external carrier comprises a magnetic dipole, with a respective opposite dipole located just beneath the subject's skin. In this case, the opposite dipole may preferably be part of the implant unit. Further means of attachment can include a hook-and-loop fastener based system (e.g. Velcro^{®}) or an ear-loop based system, which would require no skin adhesive patch. The external carrier may be flexible or rigid, or may have flexible portions and rigid portions. The external carrier and/or the housing of the external unit may further be constructed of any suitable material. In particular, the external carrier or the housing may include a flexible material such that the external unit may be configured to conform to a desired location. The material of the flexible substrate may include, but is not limited to, plastic, silicone, woven natural fibers, and other suitable polymers, copolymers, and combinations thereof. According to a preferred embodiment of the invention, the design of the external carrier may allow the skin to "breathe", e.g. by being at least partially air-permeable. Further, the external carrier may comprise a soft material, Any portion of external unit may be flexible or rigid, depending on the requirements of a particular application. The external unit may be configured to be affixed to the subject. For example, for a subject suffering from head and facial pain, the external unit may be attached behind the subject's ear at the level of the mastoid process.. Suitable locations for the external unit may be determined by communication between external unit and the implant unit. Additionally, the external unit may generate a signal that provides the subject with feedback regarding the optimal position of the external unit. The optimal position may for example be measured based on a feedback from the implantable unit to the external unit. Once a desired position is detected, a vibrator module located in the external unit may give a vibration signal indicating correct alignment of the internal unit with the external unit.

Furthermore, the carrier may include a connector for selectively or removably connecting the housing, the connector extending or protruding from the external carrier to be received by a recess of the housing. Alternatively, the housing can comprise a connector to be received by a recess of the external carrier. Preferably, the external carrier will be a mechanically flexible rectangular- or circular-shaped patch that will suit placement in the curved area behind the ear of the subject.

According to an additional embodiment, the system may further comprise a physician external unit and a physician programming unit. The physician external unit may for example be normal external unit in the sense of this disclosure, having a specialized software installed on it, wherein the specialized software is dedicated to be used by the physician. The physician programming unit may be an external computing unit, e.g. a PC or a similar handheld or portable device like a tablet or a smartphone. Together, the physician external unit and the physician programming unit may be configured to prepare a therapy template and store the configuration under specific names. Furthermore, the physician programming unit may serve for secure identification of the implant unit and establish a secure pairing between the implant unit and the physician external unit. Further functions of the physician external unit and the physician programming unit may comprise: Verifying patient history log files stored in the implant unit; prepare/load a therapy and run a test stimulation sequence during patient visits; program the tested therapy during patient visits; receive a status report from the implant unit (e.g. impedance of the electrodes, alerts or warnings, history log files, etc.) during the patient visits; provide a user-friendly interface to visualize the therapy before programming (e.g. show electrode configuration, current amplitudes, pulse durations, ON-OFF period, etc.) It is understood the data transfer between the physician external unit and the implant unit may be carried in real-time.

The modulation electrodes may further be configured to cause, when supplied with the modulation signal, a unidirectional and/or a bidirectional electrical field. For this purpose, the modulation electrodes may be made from conductive and biocompatible materials, such as stainless steel, gold, platinum, iridium, platinum-iridium, platinum-gold, conductive polymers, etc. In particular, the electrodes may be configured to facilitate, when supplied with the modulation signal, a substantially unidirectional and/or bidirectional electric field sufficient to modulate the occipital nerve. Furthermore, the modulation electrodes may include anode and cathode electrode pairs, which may be spaced apart by about a distance of about 0.2 mm to 20 mm. Other configurations, like tripolar electrodes (e.g. + - +), may be used, as well. Furthermore, monopolar electrode systems may be used, wherein the housing of the implantable unit comprises a (large) indifferent electrode. The implant may also comprise electronic components like a switch matrix to select the configuration.

The external unit may be configured to communicate with the implant unit. A circuitry of the external unit may, for example, be configured to generate an electric primary signal on the primary transmission unit that may cause an electric secondary signal on the secondary transmission unit in the implant unit. In certain embodiments, however, it may be advantageous (e.g. in order to generate a unidirectional electric field for modulation of a nerve) to provide a temporary or continuous DC (Direct Current) field inducing signal at the modulation electrodes. Additionally, a combination of AC and DC signals may be implemented with the system of this disclosure. To convert the AC secondary signal on the secondary transmission unit to a DC field inducing signal, the implant unit may include a signal modifier, for example, a demodulator or an AC-DC converter. The AC to DC converter may include any suitable converter known to those skilled in the art. For example, in some embodiments the AC-DC converter may include rectification circuit components including, for example, diodes and appropriate capacitors and resistors. In alternative embodiments, the implant unit may include an AC-AC converter, or no converter, in order to provide an AC field inducing signal at modulation electrodes.

As noted above, the secondary signal may be configured to generate an electric field between the modulation electrodes. In some instances, the magnitude, orientation, energy density, and/or duration of the generated electric field resulting from the secondary signal on the secondary transmission unit may cause current flow sufficient to modulate one or more nerves in the vicinity of the electrodes. In such cases, the secondary signal may be referred to as a modulation signal, and the associated primary signal may be referred to as a modulation control signal. In other instances, the magnitude and/or duration of the field inducing signal may generate an electric field that does not result in nerve modulation. In such cases, the field inducing signal may be referred to as a sub-modulation signal.

In some instances, the magnitude and/or duration of the generated electric field resulting from the field inducing signal may be sufficient to modulate one or more nerves in the vicinity of the modulation electrodes. In such cases, the field inducing signal may be referred to as a modulation signal. In other instances, the magnitude and/or duration of the field inducing signal may generate an electric field that does not result in nerve modulation. In such cases, the field inducing signal may be referred to as a sub-modulation signal. Various types of field inducing signals may constitute modulation signals. For example, in some embodiments, a modulation signal may include a moderate amplitude and moderate duration, while in other embodiments, a modulation signal may include a higher amplitude and a shorter duration. Various amplitudes and/or durations of field-inducing signals across electrodes may result in modulation signals, and whether a field-inducing signal rises to the level of a modulation signal can depend on many factors (e.g. distance from a particular nerve to be stimulated; whether the nerve is branched; orientation of the induced electric field with respect to the nerve; type of tissue present between the electrodes and the nerve; etc.).

According to another embodiment, the power source may be permanently or removably coupled to a location within the external unit. The power source may further include any suitable source of power configured to be in electrical communication with the processor. For example, the power source may include a rechargeable and/or replaceable battery, such as a paper battery, thin film battery or other type of battery. In some embodiments, the power source may include a substantially flat, flexible battery. The power source may provide power for the system, in particular for stimulation. In many embodiments, since the size of the external unit must be as small as possible, the total size of the external unit will be determined by the size of the power source, e.g. the battery. Preferably, the power source has an area that is smaller than or equal to 500 mm².

According to a preferred embodiment, the system includes a charging unit configured for charging the power source of the external unit. For example, it can be intended to recharge the power source of the external unit during daytime. The charging unit may comprise one or more of the following: a wire connector (e.g. a USB-Type connector) for power supply; a transmission unit or antenna for establishing an inductive link to recharge the external unit; a CPU or a memory (e.g. a microcontroller); a local area network or internet interface (e.g. a Wi-Fi-interface) configured for connection to a webserver; at least one LED; a 4G module configured for transferring information to a webserver.

Amongst the preferred functions of the external unit may be the following: recharge the rechargeable battery of the external unit; display the battery status of the external unit on a display or similar user interface; drive one or more LEDs signaling charging process; drive a LED signaling receipt of an alert; send data stored on the external unit and sent by the implant unit to a webserver using local area network or internet interface (e.g. a Wi-Fi-interface); etc.

The circuitry of the implant unit, i.e. the modulation electrodes, the electronics, the antenna and connecting wires, may include conductive and/or biocompatible materials, such gold, platinum, iridium, platinum-iridium, platinum-gold, conductive polymers, etc. Preferably, the implanted unit is substantially manufactured in one piece, using only one material (e.g. silicone), with the exception of the electronic components (i.e. electrodes, wires, transmission units, insulators etc.). Alternatively, a biological glue may be used to join the stimulation bridge and the transmission unit together.

According to another preferred embodiment, it is also possible that the lead comprises a flexible carrier, wherein the secondary transmission unit and the at least one lead are each connected to the flexible carrier sized and configured for implantation beneath the skin. In that case, the lead comprising the modulation electrodes may also be referred to as stimulation bridge. The secondary transmission unit may be mounted onto or otherwise be integrated with the flexible carrier. Further, the secondary transmission unit may include a coil antenna. Such a coil antenna may be made from any suitable conductive material and may be configured to include any suitable arrangement of conductive coils (diameter, number of coils, layout of coils, etc.). A coil antenna suitable for use as secondary transmission unit may have a diameter of about 0.5 cm to 5 cm, and may be circular-, rectangular- or oval-shaped. A coil antenna suitable for use as secondary transmission unit may have any number of windings. Further, a coil antenna suitable for use as secondary transmission unit may have a wire diameter of about 0.1 mm to 2 mm. According to another embodiment, the transmission unit can be a printed circuit board antenna.

In some embodiments, the flexible carrier of the implant comprises a flexible, biocompatible, material and/or an insulating material. Such materials may include, for example, silicone, phenyltrimethoxysilane (PTMS), polymethyl methacrylate (PMMA), Parylene C, polyimide, liquid polyimide, laminated polyimide, black epoxy, polyether ether ketone (PEEK), Liquid Crystal Polymer (LCP), Kapton, etc. Further to the above, the implant may be encapsulated in at least one layer of a biocompatible material, and may include ceramic material, thermoplastic material such as ULTEM, or other compatible materials.

Since the implant unit does not comprise a power source (e.g. in form of a battery), it can be kept small. Thus, it can be implanted through a short and minimally invasive procedure. In particular, a system according to this disclosure may be implanted in a one-day hospital setting that should not last more than 30 minutes. The flexible carrier may also be fabricated with a thickness suitable for implantation under a patient's skin. For example, the implant unit may have thickness of less than 4 mm, in particular of less than about 2 mm. In a preferred embodiment, the implant is configured for implantation through a 3 cm incision in the subject's skin at the level of the mastoid process.

In addition to the above, the modulation electrodes may be located alongside the at least one lead. The lead comprises modulation electrodes and may preferably be configured for implantation in the vicinity of one or more nerves to be modulated such that the electrodes are spaced apart from one another along a longitudinal direction of a number nerves. It may be beneficial if one of the modulation electrodes is located at one of the lead's ends. For example, modulation electrodes may be spaced apart by a distance of about 1 mm to 20 mm, or between 5 mm and 10 mm. In a preferred embodiment, the at least one lead may comprise eight modulation electrodes evenly spaced along the lead ("octopolar lead"). The electrodes may further be configured to facilitate an electric field in response to an applied electric signal, the electric field including field lines extending in the longitudinal direction of the nerve to be modulated.

The at least one lead and the modulation electrodes may form a stimulation bridge. A stimulation bridge according to this embodiment has an elongated shape. Preferably, the length of the stimulation bridge is at least 20 times the width or diameter of the stimulation bridge. According to an even preferred embodiment, the length of the stimulation bridge is at least 50 times the width or diameter of the stimulation bridge. For example, the lead may have a diameter of 1-3 mm and a length of up to 100 mm. Once implanted, the at least one stimulation bridge may be located in such a way inside the subject's body that it is directed towards a point of about 1 to 1.5 cm above the subject's inion. Preferably, the at least one stimulation bridge comprises a modulation electrode at the end of the lead that has a maximum distance from the secondary transmission unit. The stimulation bridge may in particular be configured for implantation in such a way that at least one modulation electrode is located along the lead close to the secondary transmission unit, which may be mounted on the flexible carrier. The secondary transmission unit may then be located in the vicinity of the C2 vertebra, as this is the link of the trigeminal cervical complex and the dorsal root ganglion of C2. The precise location of the secondary transmission unit of the implant unit also determines the position of the external unit when attached to the subject's skin.

With a stimulation bridge as described herein, it is possible to precisely innervate or stimulate a single nerve, as opposed to only being able to stimulate a greater neural area. Obviously, the latter is still possible in addition to single nerve stimulation by means of the stimulation bridge as described.

Preferably, the lead of the stimulation bridge is oval-, rectangular- or lens-shaped when viewed in a transversal cross-section, wherein the electrodes are only disposed on the side of the lead that is facing the nerve to be stimulated. This way, the stimulation signal can be focused in a much more precise manner, which in tune is more energy-efficient. A stimulation bridge having an oval or lens-shape when viewed in a cross-section has the additional advantage that its rounded "edges" may lead to a higher comfort for the subject or patient once implanted.

According to a preferred embodiment, the lead or the stimulation bridge may be configured for bilateral stimulation or for unilateral stimulation, wherein unilateral stimulation comprises a short lead and bilateral stimulation comprises a long lead. For example, it may be intended that the short lead (unilateral stimulation) is shorter than 10 cm or about 10 cm of length, whereas the long lead (bilateral stimulation) is longer than 10 cm. A stimulation bridge configured for bilateral stimulation may for example comprise a total of 16 electrodes (i.e. two distinct sections each comprising 8 modulation electrodes)

Further, bilateral stimulation requires two incisions, one small (3 cm) incision at the level of the mastoid process and a second one 1 to 1.5 cm above the inion for implantation of the stimulation bridge. However, according to a preferred embodiment, only one channel will be required for both forms of stimulation (unilateral and bilateral). With a stimulation bridge having a lead that is longer than 10 cm, i.e. that is configured for bilateral stimulation, it is possible to stimulate a larger neural area of the subject with only to minor incisions. Thus, the medical procedure of implanting the implant unit can be kept relatively short and still allow for a very efficient neural stimulation.

The processor may comprise programmable electronics and be configured to cause transmission of a modulation signal from the primary transmission unit to the secondary transmission unit of the implant unit implanted beneath the skin of the subject, i.e. beneath the subcutaneous tissue. The processor may be further configured to adjust one or more characteristics of the modulation signal to generate a sub-modulation control signal adapted to cause a current at the at least modulation electrode below a neuronal modulation threshold of the occipital nerve when received by the secondary transmission unit of the implant unit and to generate the modulation signal adapted to cause a current at the modulation electrode above a neuronal modulation threshold of the occipital nerve when received by the secondary transmission unit of the implant unit. This way, the external unit can generate a magnetic field by radiofrequency, which in turn generates stimulation of the occipital nerve field through the modulation electrodes of implant unit. Thus, there is no longer a need for having an implant pulse generator (IPG) implanted deep in the muscle or to make loops, excess tunneling or to add extensions all the way down to the IPG, which can take much time during the respective implantation procedure. Preferably, the distance between the external unit and the (implanted) implant unit is less than 5 cm, in particular less than 1 cm.

According to certain embodiment, the processor may be programmable to cause transmission of a modulation signal from the primary transmission unit to the secondary transmission unit of the implant unit based on distinct pre-programmed modulation protocols (including one or more "modes"). For example, a "night mode" protocol may be provided, the protocol including:
- decreasing the voltage or current amplitude of the modulation signal in a specific range or percentage;
- adding OFF periods to the duty cycle
- switching to a "burst mode" instead of a "tonic mode".

Burst stimulation typically delivers groups of pulses at a higher frequency and at amplitudes much lower than tonic stimulation and is considered non-detectable by a patient and thus well indicated overnight. This way, effective stimulation can be maintained during the sleep of a patient, without being interruptive or otherwise uncomfortable.

The at least one processor may include any electric circuit that may be configured to perform a logic operation on at least one input variable. The at least one processor may therefore include one or more integrated circuits, microchips, microcontrollers, and microprocessors, a digital signal processor (DSP), a field programmable gate array (FPGA).

According to a further embodiment, the external unit may comprise a storage unit in electrical connection with the processor, configured to store stimulation data and. Such data can comprise any form physiological parameter of the subject, e.g. feedback of the subject and stimulation parameter. This way, the processor can perform predictive stimulation control on based on patient feedback, i.e. based on the stored physiological parameters.

Further, the external unit may comprise an energy harvesting unit configured for deriving energy from an external source. The external source may be solar power, thermal power (e.g. in the form of the subject's body heat), kinetic energy (due to movement of the subject) or the like. The energy harvesting unit may further be connected to the power source of the external unit, thereby recharging said power source.

As part of a preferred embodiment, the implant unit may comprise tines or spikes for fixation of the at least one lead to a tissue of the subject. This way, dislocation of the implant unit from the tissue can be avoided, which would otherwise occur due to neck movements of the subject. The tines can be disposed on the flexible carrier and/or on the at least one lead of the implant unit and may preferably be made from a biocompatible material. According to another embodiment, fixation of the main body of the implant unit may comprise meshes. It is also possible that RX markers are placed along the lead to verify correct placement during the surgery procedure by use of an RX machine and a screen for the lead and RX markers visualization.

It is further possible that the implant unit is configured implantation in a substantial hairless region of the head to optimize the communication between external and internal unit. Modulation of the occipital nerves, such as the greater, lesser and third occipital nerve, and secondary the trigemino cervical complex will be possible through the stimulation bridge and will be useful for treating head and facial pain, such as that from migraines. It may be intended not to stimulate deep brain structures and spinal cord. The neurostimulation may instead be focused on chronic migraine (CM) and chronic cluster headache (CCH), refractory to pharmacological treatments. For those patients, especially for CCH, there is still an unmet medical need.

The system further may comprise a remote control configured for adjustment of the neurostimulation. The remote control enables the subject to self-adjust the electrical stimulation of the respective nerve. This is especially useful for patients with chronic migraine and chronic cluster headache (CCH), which require continuous stimulation. In certain embodiments, the remote control can be in the form of a dedicated software ("App") to be installed on a computer or on any suitable handheld device (e.g. on a mobile phone or on a tablet).

The coupling between the secondary transmission unit of the implant unit and the primary transmission unit of the external unit can comprise capacitive coupling, inductive coupling, ultrasound, light and/or radiofrequency coupling. In particular, such coupling of the primary transmission unit and the secondary transmission unit may include any interaction between the primary transmission unit and the secondary transmission unit that causes a signal on the secondary transmission unit in response to a signal applied to the primary transmission unit, wherein each signal may comprise power and/or data. In some embodiments, coupling between the primary and secondary transmission units may include capacitive coupling, inductive coupling, radiofrequency coupling, etc. and any combination thereof. As a result of coupling between the primary transmission unit and the secondary transmission unit, a secondary signal may arise on the secondary transmission unit when the primary signal is present on the primary transmission unit. Such coupling may include inductive/magnetic coupling, RF coupling/transmission, capacitive coupling, or any other mechanism where a secondary signal may be generated on the secondary transmission unit in response to a primary signal generated on the primary transmission unit.

According to another embodiment, the implant unit may comprise a processor in addition to the processor comprised by the external unit. The processor of the implant unit may then be referred to as internal processor. When implemented, the internal processor may comprise at least one software program installed on it. Thus, the internal processor may be configured to run and diagnose stimulation therapy and send alerts and warnings (A&W) to the external unit in case the implant unit encounters problems.

As a preferred embodiment, a system for treating head and facial pain could comprise the external unit be attached to the external carrier which is attached to a patient's skin. This "patient external unit" may establish, via its primary transmission unit, a transmission link with the implant unit implanted beneath the subject's skin. Furthermore, the external unit may supply power to the implant unit via induction. The implant unit may then be configured to communicate with the external unit via wireless transmission and may further comprise a bootloader which starts a software program installed on the internal processor of the implant unit. Lastly, the implant unit may be configured to run and diagnose stimulation therapy and send alerts and warnings (A&W) to the external unit in case the implant unit encounters problems. The system may further comprise a charging unit for charging the "patient external unit" via inductive coupling. However, the charging unit may further be configured to collect A&W data stored on the external unit and sent to the external unit from the implant unit. Furthermore, the charging unit may comprise a charger LED and an emergency LED, which can be turned on or off. For example, the charger LED may be turned on for as long as the external unit is being charged by the charging unit. Likewise, the emergency LED may be turned on when the charging unit receives A&W data from the external unit. Additionally, the charging unit may connect to a web server and upload a log file and an A&W report (if present).

Additionally, the preferred system may comprise a physician programming unit connectable to the external unit. When the external unit is connected to the physician programming unit, it may also be referred to as "physician external unit". The "physician external unit" may be the same device as the "patient external unit" or it may be a different external device used solely by the physician. The physician programming unit may be an external computing unit, e.g. a PC or a similar handheld or portable device like a tablet or a smartphone. Together, the physician external unit and the physician programming unit may be configured to prepare a therapy template and store the configuration under specific names. Furthermore, the physician programming unit may serve for secure identification of the implant unit and establish a secure pairing between the implant unit and the physician external unit. Further functions of the physician programming unit and the physician programming unit may comprise: Verifying patient history log files stored in the implant unit; prepare/load a therapy and run a test stimulation sequence during patient visits; program the tested therapy during patient visits; receive a status report from the implant unit (e.g. alerts or warnings, history log files, etc.) during the patient visits; provide a user-friendly interface to visualize the therapy before programming (e.g. show electrode configuration, current amplitudes, pulse durations, ON-OFF period, etc.).

According to another aspect of the present disclosure, an implant unit for use in a neurostimulation system according to any of the preceding claims is presented, wherein the implant unit is configured for implantation inside a subject's body through an incision the subject's skin, and wherein the implant unit is further configured for implantation inside the subject's body through a tunnel in the subject's fat tissue leading from the incision. Preferably, the incision is 0.5 to 3.5 cm long. Furthermore, the implant unit may be configured for implantation inside a tunnel in the subject's fat tissue or subcutaneous tissue, wherein the tunnel leads from the incision toward a location 2-3 cm above the inion covering the occipital area.

According to yet another aspect of the disclosure, a method for electrical stimulation of neuromuscular tissue using a neurostimulation system is described herewith, the method comprising: sending stimulation parameters from an external unit to an implant unit; generating an electrical stimulation pattern with the external unit, the electrical stimulation pattern comprising at least one modulation signal; delivering the electrical stimulation pattern to an implant unit located inside a subject's body; adjusting the electrical stimulation pattern, wherein adjusting the electrical stimulation pattern comprises changing of electrode configuration and increasing or decreasing a voltage, a current amplitude, a pulse frequency and/or a pulse width of the electrical stimulation pattern. Such a method may be valuable, for example, in pain management, where the propagation of pain signals is undesired. Preferably, in order to reduce habituation in the patient or subject, the method can include one or more stochastic elements. For example, the stimulation parameters may comprise a stochastic behavior in a defined range, such that the voltage, the current amplitude, the pulse frequency and/or the pulse width of the electrical stimulation pattern may vary in a stochastic manner. Further, the electrodes within an electrode configuration may stochastically innervate different neural areas at different times, thereby generating random stimulation patterns.

The method may further include receiving an alternating current (AC) signal at a secondary transmission unit of the implant unit, the implant unit generating a voltage signal in response to the AC signal. The method may further include applying the voltage signal to at least one pair of modulation electrodes configured for implantation in the vicinity of the nerve to be modulated.

### Brief Description of the Drawings

The accompanying drawings, which are incorporated in and constitute a part of this specification, illustrate several examples of the disclosed subject matter. The drawings depict the following:
Fig. 1 depicts a schematic back view of a subject with a system for unilateral neurostimulation, comprising an implant unit and an external unit according to an exemplary embodiment of the present disclosure;
Fig. 2 depicts a schematic back view of a subject with a system for bilateral neurostimulation, comprising an implant unit and an external unit according to an exemplary embodiment of the present disclosure.
Fig. 3 depicts a schematic view of an exemplary embodiment of an implant unit comprising a unilateral modulation electrode configuration.
Fig. 4 depicts a schematic view of an exemplary embodiment of an implant unit comprising a bilateral modulation electrode configuration.
Fig. 5 depicts a flowchart of a system for treating head and facial pain according to an exemplary embodiment.

### Detailed Description of the Exemplary Embodiments

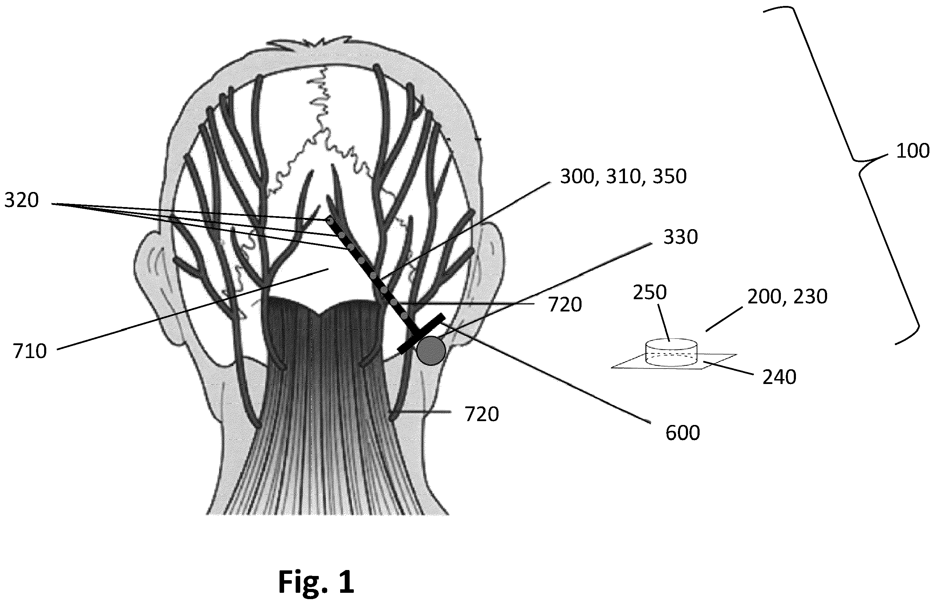

Fig. 1 depicts a schematic back view of a subject with a system 100 for unilateral neurostimulation, the system comprising an implant unit 300 and an external unit 200 according to an exemplary embodiment of the present disclosure. The external unit 200 is configured for location external to the subject 700 or patient. The external unit 200 as shown in Fig. 1 is not attached to the subject. However, it may in fact be configured to be affixed to the subject 700. For example, for a subject suffering from head pain, the external unit 200 may be attached in the vicinity of the mastoid process, approximately at the level of the C2 vertebra. Suitable locations for the external unit 200 may be determined by communication between external unit 200 and the implant unit 300 implanted in the subject. The external unit 200 may comprise an external carrier 240 configured for attachment to the subject's 700 skin. For example, the carrier 240 may be a flexible skin patch configured for adherence to the subject's 700 skin, e.g. through adhesives or mechanical means. It is also possible to attach the external unit 200 to the subject's 700 skin via magnetic force, wherein the external carrier 240 comprises a magnetic pole, with a respective opposite pole located just beneath the subject's 700 skin. Further means of attachment can include a Hook-and-loop fastener based system (e.g. Velcro^{®}). The external carrier 240 may be flexible or rigid, or may have flexible portions and rigid portions. The external carrier 240 and/or the housing 250 of the external unit 200 may be constructed of any suitable material. In particular, the external unit or the housing 250 may include a flexible material such that the external unit may be configured to conform to a desired location. The material of the flexible substrate may include, but is not limited to, plastic, silicone, woven natural fibers, and other suitable polymers, copolymers, and combinations thereof. Any portion of external unit may be flexible or rigid, depending on the requirements of a particular application.

The external unit 200 may further be configured to be affixed to an alternative location proximate to the subject. For example, in one embodiment, the external unit 200 may be configured to fixedly or removably adhere to a strap or a band that may be configured to wrap around a part of a subject's body. Alternatively, or in addition, the external unit 200 may be configured to remain in a desired location external to the subject's body without adhering to that location.

The external unit 200 may comprise a housing 250. The housing 250 may include any suitable container configured for retaining components, e.g. a primary transmission unit 230 or a processor 210. In addition, while the external unit 200 is illustrated schematically in Fig. 1, the housing 250 may be any suitable size and/or shape and may be rigid or flexible.

The primary transmission unit 230 may be in the form of a coil antenna having a diameter of about 0.5 cm to 5 cm, and may preferably be circular-shaped or oval-shaped. A coil antenna suitable for use as primary transmission unit may have any number of windings. Further, a coil antenna suitable for use as primary transmission unit may have a wire diameter of about 0.1 mm to 2 mm.

As previously discussed, the external unit 200 may be configured to adhere to a desired location. Accordingly, in some embodiments, at least one side of the housing 250 or external carrier 240 may include an adhesive material. The adhesive material may include a biocompatible material and may allow for a subject to attach the external unit 200 to the desired location and remove the external unit 200 upon completion of use. The adhesive may be configured for single or multiple uses of the external unit 200. Suitable adhesive materials may include, but are not limited to biocompatible glues, starches, elastomers, thermoplastics, and emulsions.

Further, the external unit 200 may be associated with a power source 220. The power source 220 may be removably couplable to the external unit 200 at an exterior location relative to external unit. Alternatively, the power source 220 may be permanently coupled to the external unit 200. If the power source 220 is permanently coupled to external unit 200, it is intended that the power source 220 be rechargeable. The power source 220 may further include any suitable source of power configured to be in electrical communication with the processor. In one embodiment, for example the power source 220 may include a battery 221.

The system of Fig. 1 further discloses an implant unit 300 comprising a stimulation bridge 350, the stimulation bridge 350 including a lead 310, the secondary transmission unit 330 and pairs of modulation electrodes 320. The lead 310 in Fig. 1 is approximately 10 cm long and comprises eight pairs of modulation electrodes 320 ("octopolar lead"), evenly spaced apart along the lead 310. Further, the implant unit 300 is battery-free, making it possible for the implant unit 300 to be in the form of the stimulation bridge 350 shown in Fig. 1. The implant unit 300 is therefore suitable for use in a neurostimulation system 100 as disclosed herein, since it is configured for implantation inside a subject's 700 body through an incision 600 the subject's 700 skin, wherein the incision is 0.5 to 3.5 cm long. Furthermore, the implant unit 100 may be configured for implantation inside a tunnel 610 in the subject's tissue, wherein the tunnel 610 leads from the incision 600 towards a location in the vicinity of the subject's 700 inion 710, e.g. in a vicinity of an occipital nerve 720.

Upon coupling of the primary transmission unit 230 of the external unit 200 with the secondary transmission unit 330 of the implant unit 300, a sub-modulation signal or a modulation signal may be transmitted from the external unit 200 to the modulation electrodes 320 of the implant unit 300. A circuitry of the external unit 200 may for example be configured to generate an electric primary signal on the primary transmission unit 230 that may cause an electric secondary signal on the secondary transmission unit 330 in the implant unit 300. The secondary signal may then be configured to generate an electric field between the modulation electrodes 320, sufficient to modulate the terminal fibers of a nerve, when spaced apart thereof.

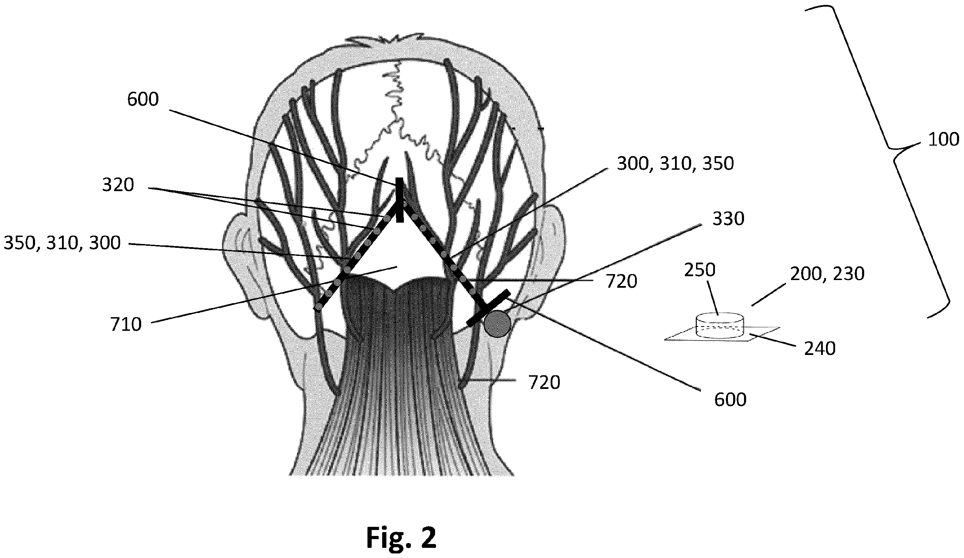

Fig. 2 depicts a schematic back view of a subject with a system 100 for bilateral neurostimulation, comprising an implant unit 300 and an external unit 200 according to an exemplary embodiment of the present disclosure. The embodiment shown in Fig. 2 differs from that of Fig. 1 in that a long lead 310 or stimulation bridge 350 is used, which is suitable for bilateral stimulation. The lead 310 of the stimulation bridge 350 shown in Fig. 2 is approximately 20 cm long. Preferably, it is configured for an implantation procedure using two incisions 600.

In particular, bilateral neurostimulation requires one small incision 600 of 3 cm length at the level of the mastoid process and a second incision 600, the second incision being located 1 to 1.5 cm above the inion for implantation of the stimulation bridge 350. With a stimulation 350 bridge having a lead 310 that is longer than 10 cm, i.e. that is configured for bilateral stimulation, it is possible to stimulate a large neural area of the subject 700 with only to minor incisions 600. Thus, the medical procedure of implanting the implant unit in the subject 700 can be kept short and still allow for a very efficient neural stimulation.

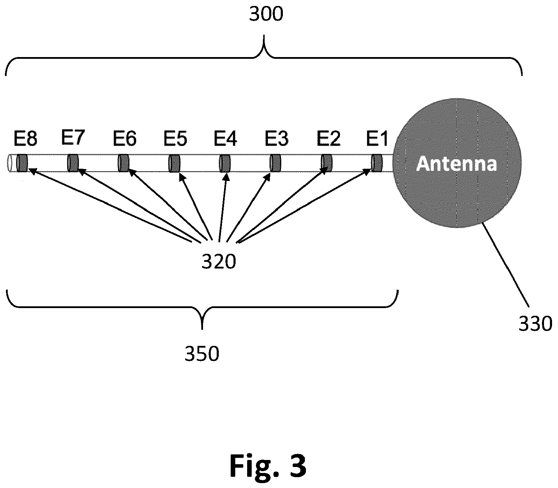

Fig. 3 depicts a schematic view of an exemplary embodiment of an implant unit 300 comprising a unilateral modulation electrode 320 configuration. In particular, the implant unit 300 of Fig. 3 is composed of the stimulation bridge 350 the secondary transmission unit 330. The stimulation bridge 350 comprises a lead 310 of approximately 10 cm of length as well as and eight pairs of modulation electrodes 320 (also labeled E1 to E8 in Fig. 3) attached to the lead 310 ("octopolar lead"). According to the embodiment shown in Fig. 3, the modulation electrodes 320 are evenly spaced apart along the lead 310.

The implant unit 300 of Fig. 3 is battery-free, making it possible for the implant unit 300 to be in the slim and relatively small form of the stimulation bridge 350 shown in Fig. 3. Such an implant unit 300 is suitable for use in a neurostimulation system 100 as disclosed herein, since it is configured for implantation inside a subject's 700 body through an incision 600 the subject's 700 skin, wherein the incision is 0.5 to 3.5 cm long. Furthermore, the implant unit 300 may be configured for implantation inside a tunnel 610 in the subject's 700 tissue, wherein the tunnel 610 leads from the incision 600 towards a location in the vicinity of the subject's 700 inion 710, e.g. in a vicinity of an occipital nerve 720.

In the electrode 320 configuration of the stimulation bridge 350 of Fig. 3, any individual modulation electrode E1 to E8 can be programmed as an Anode (A) or a Cathode (C) or it can be left unconnected (NC). According to a preferred embodiment, at least one electrode 320 may be defined as Anode and/or at least one electrode 320 may be defined as Cathode. The remaining to two to seven electrodes may be put in parallel as Anode or Cathode.

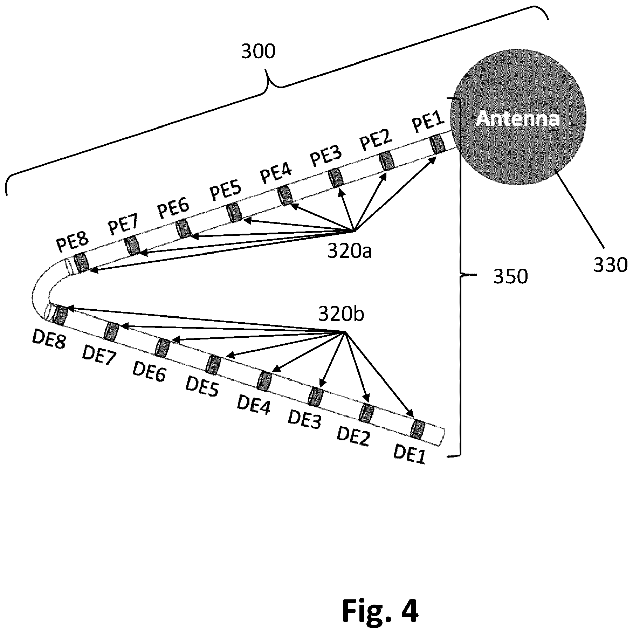

Fig. 4 depicts a schematic view of an exemplary embodiment of an implant unit 300 comprising a bilateral modulation electrode 320 configuration. The implant unit 300 of Fig. 4 differs from the embodiment of Fig. 3 in the stimulation bridge 350 comprises a longer lead 310, suitable for bilateral stimulation. The lead 310 of the stimulation bridge 350 shown in Fig. 3 is approximately 20 cm long.

As shown on Fig. 4, the stimulation bridge 350 is made up of two connected segments, each segment comprising eight pairs of modulation electrodes 320a and 320b respectively. The modulation electrodes 320a attached to the segment of the stimulation bridge 350 that is connected to the secondary transmission unit 330 are called proximal electrodes 320a (also labeled PE1 to PE8 in Fig. 4). Likewise, the modulation electrodes 320b attached to the segment of the stimulation bridge 350 that is not directly connected to the secondary transmission unit 330 are called distal electrodes 320b (also labeled DE1 to DE8 in Fig. 4).

According to the electrode 320 configuration of the stimulation bridge 350 of Fig. 4, any individual modulation electrode PE1 to PE8 or DE1 to DE8 can be programmed as an Anode (A) or a Cathode (C) or it can be left unconnected (NC). According to a preferred embodiment, at least one electrode 320 may be defined as Anode and/or at least one electrode 320 may be defined as Cathode.

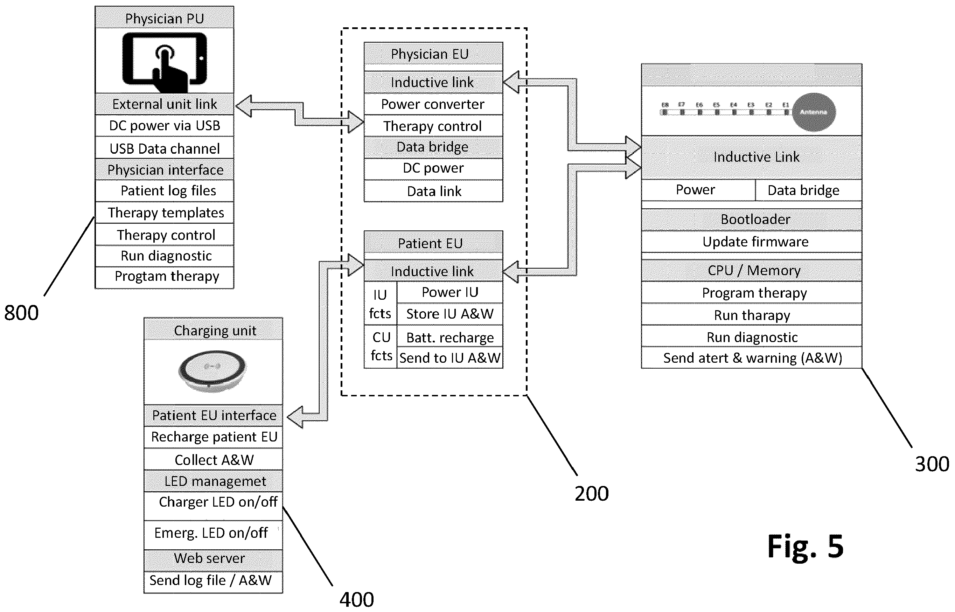

Fig. 5 depicts a flowchart of a system 100 for treating head and facial pain according to an exemplary embodiment. According to this embodiment, an external unit 200 may be attached to the external carrier 240 which is attached to a patient's skin. This "patient external unit" 200 (Patient EU in Fig. 5) establishes, via its primary transmission unit 230, a transmission link with an implant unit 300 implanted beneath the patient's 700 skin. Furthermore, the "patient external unit" 200 may supply power to the implant unit 300 via induction.

According to the shown embodiment of the system 100, the implant unit 300 is configured to communicate with the external unit 200 via wireless transmission. The implant unit may further comprise a bootloader which starts a software program installed on a processor of the implant unit. Lastly, the implant unit 300 may be configured to run and diagnose stimulation therapy and send alters and warnings (A&W) to the external unit 200 in case the implant unit 300 encounters problems.

The system 100 further comprises a charging unit 400 for charging the external unit 200 via inductive coupling. However, according to the embodiment of Fig. 5, the charging unit 400 may further be configured to collect A&W data stored on the patient external unit 200 and sent to the external unit 200 from the implant unit 300. Furthermore, the charging unit 400 may comprise a charger LED and an emergency LED, which can be turned on or off. E.g. the charger LED may be turned on for as long as the external unit 200 is being charged. Likewise, the emergency LED may be turned on when the charging unit 400 receives A&W data from the external unit 200. Additionally, the charging unit 400 may connect to a web server and upload log files or A&W reports.

Lastly, as shown by Fig. 5 the system 100 may comprise a physician programming unit 800 connectable to the external unit 200. When the external unit 200 is connected to the physician programming unit 800, it may also be referred to as "physician external unit" 200 (see Physician EU in Fig. 5). The "physician external unit" 200 may then either be the same device as the "patient external unit" (see Patient EU in Fig. 5) or it may be a different external device used solely by the physician. The physician programming unit 800 may be an external computing unit, e.g. a PC or a similar handheld or portable device like a tablet or a smartphone. Together, the physician external unit 200 and the physician programming unit 800 are configured to prepare a therapy template and store its configuration under specific names. Furthermore, the physician programming unit 800 may serve for secure identification of the implant unit 300 and establish a secure pairing between the implant unit 300 and the physician external unit 200. Further functions of the physician programming unit 800 and the physician programming unit 200 may comprise: Verifying patient history log files stored in the implant unit 300; prepare/load a therapy and run a test stimulation sequence during patient visits; program the tested therapy during patient visits; receive a status report from the implant unit 300 (e.g. alerts or warnings, history log files, etc.) during the patient visits; provide a user-friendly interface to visualize the therapy before programming (e.g. show electrode configuration, current amplitudes, pulse durations, ON-OFF period, etc.).

The invention is not limited to one of the embodiments described herein but may be modified in numerous other ways.

All features disclosed by the claims, the specification and the figures, as well as all advantages, including constructive particulars, spatial arrangements and methodological steps, can be essential to the invention either on their own or by various combinations with each other.

### List of reference numerals

- 100: System

- 200: External unit
- 210: Processor
- 220: Power source
- 221: Battery
- 230: Primary transmission unit
- 240: External carrier
- 250: Housing
- 260: Energy harvesting unit
- 300: Implant unit
- 310: Lead
- 320: Modulation electrodes
- 330: Secondary transmission unit
- 340: Flexible carrier
- 350: Stimulation bridge
- 400: Charging unit
- 500: Remote control
- 600: Incision
- 610: Tunnel
- 700: Subject
- 710: Inion
- 720: Occipital nerve
- 800: Physician programming unit

## Claims

1. A neurostimulation system (100) for treating head and facial pain, the system (100) comprising:
an external unit (200) configured for a location external to the subject's (700) body;
an implant unit (300) configured for implantation inside a subject's (700) body; and
wherein the external unit (200) comprises:
a processor (210);
a power source (220); and
a primary transmission unit (230) in electrical communication with the power source (220) and the processor (210);
wherein the implant unit (300) comprises:
at least one lead (310);
at least one pair of modulation electrodes (320) attached to the at least one lead (310); and
a secondary transmission unit (330) in electrical communication with the at least one lead (310) and the modulation electrodes (320); and
wherein the processor (210) is configured to establish a coupling between the primary transmission unit (230) and the secondary transmission unit (330) and to transmit power and/or data from the power source (220) to the implant unit (300) via said coupling.

2. The system (100) of claim 1, **characterized in that** the system (100) comprises a charging unit (400).

3. The system (100) according to any one of the preceding claims, **characterized in that** the modulation electrodes (320) are located along the at least one lead (310), wherein the electrodes (320) are in particular spaced evenly along the lead (310).

4. The system (100) according to any one of the preceding claims, **characterized in that** the implant unit (300) comprises tines (350) for fixation of the at least one lead (310) to a subcutaneous tissue of the subject (700).

5. The system (100) according to any one of the preceding claims, **characterized in that** the external unit (200) is configured for attachment to the subject's (700) skin.

6. The system (100) according to any one of the preceding claims, **characterized in that** the external (200) unit comprises an energy harvesting unit (260) configured for deriving energy from an external source.

7. The system (100) according to any one of the preceding claims, **characterized in that** the power source (220) comprises a battery (221).

8. The system (100) according to any one of the preceding claims, **characterized in that** the charging unit (400) is configured for charging the battery (221) of the external unit (200).

9. The system (100) according to any one of the preceding claims, **characterized in that** the system (100) further comprises a remote control (500) configured for adjustment of the neurostimulation.

10. The system (100) according to any one of the preceding claims, **characterized in that** the coupling between the primary transmission unit (230) and the secondary transmission unit (330) comprises capacitive coupling, inductive coupling, light, ultrasound and/or radiofrequency coupling.

11. An implant unit (300) for use in a neurostimulation system (100) according to any of the preceding claims,
wherein the implant unit (300) is configured for implantation inside a subject's (700) body through an incision (600) in the subject's (700) skin, and
wherein the implant unit (300) is further configured for implantation inside a tunnel (610) in the subject's (700) tissue.

12. The implant unit (300) of claim 13, **characterized in that** the implant unit (300) is configured for implantation through an incision (600) of 0.5 to 3.5 cm in the subject's (700) skin, and wherein the implant unit (300) is further configured for implantation inside a tunnel (610) in the subject's (700) tissue leading from the incision (600) toward a location in the vicinity of the subject's (700) inion (710).

13. The implant unit (300) according to claim 13 or 14, **characterized in that** the implant unit (300) is configured for location in a vicinity to an occipital nerve (720).

14. A method for electrical stimulation of neuromuscular tissue using a neurostimulation system (100) according to any of the preceding claims, the method comprising:
sending stimulation parameters from an external unit (200) to an implant unit (300);
generating an electrical stimulation pattern with the internal unit (300), the electrical stimulation pattern comprising at least one modulation signal;
delivering the electrical stimulation pattern to an implant unit (300) located inside a subject's (700) body;
adjusting the electrical stimulation pattern, wherein adjusting the electrical stimulation pattern comprises changing the electrode configuration and increasing or decreasing a voltage, a current amplitude, a pulse frequency and/or a pulse width of the electrical stimulation pattern.
